(19) Europäisches Patentamt
European Patent Office
Office européen des brevets

(11) EP 1 216 700 A1

(12) **EUROPÄISCHE PATENTANMELDUNG**

(43) Veröffentlichungstag:
**26.06.2002 Patentblatt 2002/26**

(51) Int Cl.[7]: **A61K 9/70**, A61K 31/42

(21) Anmeldenummer: **00250450.4**

(22) Anmeldetag: **21.12.2000**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(71) Anmelder: **Schering Aktiengesellschaft**
**13353 Berlin (DE)**

(72) Erfinder:
- **Günther, Clemens, Dr.**
**13357 Berlin (DE)**
- **Lipp, Ralph, Dr.**
**14149 Berlin (DE)**
- **Windt-Hanke, Fred**
**12157 Berlin (DE)**

(54) **Transdermalsysteme (TDS) enthaltend (R)-(-)-Methylphenyloxazolidinon-Derivate, Inhibitoren der Phosphodiesterase IV**

(57) Transdermalsystem gekennzeichnet durch einen Gehalt eines Phosphodiesterase IV-Inhibitors, insbesondere (R)-(-)-5-(4-Methoxyphenyl-3-propoxy)-5-methyl-2-oxazolidinon.

EP 1 216 700 A1

## Beschreibung

Die vorliegende Erfindung betrifft Transdermalsysteme, die (R)-(-)-Methylphenyloxazolidinon-Derivate, insbesondere (R)-(-)-5-(4-Methoxyphenyl-3-propoxy)-5-methyl-2-oxazolidinon (Mesopram (INN)) enthalten. (R)-(-)-Methylphenyloxazolidinon-Derivate sind Inhibitoren der Phosphodiesterase IV zur Behandlung immunologisch oder entzündlich verursachter Erkrankungen, insbesondere Erkrankungen des Immunsystems, die durch Stimulation von TNF und anderen Cytokinen ausgelöst werden.

Derartige Erkrankungen sind beispielsweise Autoimmunerkrankungen, pulmonare Erkrankungen, infektiöse Erkrankungen und Knochenresorptionserkrankungen, wie Rheumatoide Arthritis, Rheumatoide Spondylitits, Osteoarthritis, Gicht, Sepsis, septischer Schock, EndotoxinSchock, Grammegative Sepsis, Toxisches Schocksyndrom, Akutes Atemnotsyndrom, Pulmonare Sarkoidose, Asthma, Silikose, Kachexie, Colitis ulcerosa, Morbus Crohn, Osteoporose, Organschädigung nach Reperfusion, inflammatorische Erkrankungen des ZNS wie Cerebrale Malaria, Multiple Sklerose, Panencephalitis, Infektionserkrankungen wie AIDS, Rinderwahnsinn, inflammatorische Erkrankungen der Haut wie Urticaria, Psoriasis, Atopische Dermatitis, Kontaktdermatitis, Lupus Erythematosus sowie Diabetes Insipidus sowie Neuroprotection z. B. bei Morbus Parkinson oder Demenz nach Multiinfarkten oder Schlaganfall.

Die vorliegende Erfindung betrifft die Verwendung von (R)-(-)-Methylphenyloxazolidinon-Derivaten der Formel I,

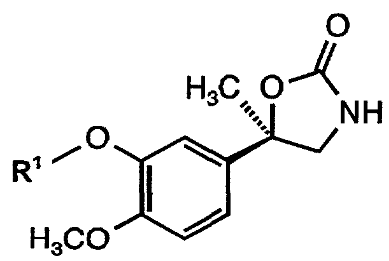

wobei $R^1$ einen Kohlenwasserstoffrest mit 1 bis 5 Kohlenstoffatomen bedeutet.

Die WO 97/15561 offenbart die Eignung von Methylphenyloxazolidinon-Derivaten zur Behandlung von Krankheiten, die durch TNF vermittelt werden und durch die auch andere Cytokine, beispielsweise Interleukin-1 oder -6, beeinflusst werden. Es werden Herstellungsverfahren für enantiomerenreine Methylphenyloxazolidinon-Derivate angegeben, wobei insbesondere das R-Derivat im Vergleich mit dem Razemat ein wirksamerer Inhibitor der Phosphodiesterase IV ist. Die zerebrale Wirkung wurde an Ratten nach intraperitonealer Verabreichung beobachtet, wobei sich das R-Enantiomer als die wirksamere Substanz erwies.

Als Verabreichungsformen werden enterale oder parenterale Formulierungen vorgeschlagen, die oral, sublingual bzw. intramuskulär oder intravenös oder aber topisch oder intrathekal verabreicht werden können.

Gegenüber diesem bekannten Stand der Technik stellt sich die vorliegende Erfindung die Aufgabe, einfach zu verabreichende, kristallfreie transdermale Formulierungen von derartigen Phosphodiesterase IV-Inhibitoren, insbesondere für (R)-(-)-5-(4-Methoxyphenyl-3-propoxy)-5-methyl-2-oxazolidinon (Mesopram (INN)) bereitzustellen, die therapeutisch wirksame Hautflüsse bei einer Pflastergröße von kleiner 50 $cm^2$ zulassen und mit denen plateauartige Plasmaspiegel erzielt werden können. Dies ist insbesondere für (R)-(-)-5-(4-Methoxyphenyl-3-propoxy)-5-methyl-2-oxazolidinon (Mesopram (INN)) wichtig, da dieser Wirkstoff eine geringe therapeutische Breite besitzt.

Die vorliegende Erfindung löst diese Aufgabe, indem sie Transdermalsysteme bereitstellt, die geeignet sind (R)-(-)-5-(4-Methoxyphenyl-3-alkoxy)-5-methyl-2-oxazolidinon-Derivate in die Haut eines Träger, insbesondere eines Menschen, so abzugeben, dass therapeutisch nutzbare Hautflüsse resultieren. Die erfindungsgemäßen Transdermalsysteme zeichnen sich durch eine spezielle Auswahl an Formulierungskomponenten aus, insbesondere des Klebers, Penetrationsverstärkers und/oder Kristallisationsinhibitors. Das erfindungsgemäße Transdermalsystem ist besonders geeignet für (R)-(-)-5-(4-Methoxyphenyl-3-propoxy)-5-methyl-2-oxazolidinon (Mesopram (INN).

Die erfindungsgemäßen Transdermalsysteme umfassen einen für den Phosphodiesterase-IV-Inhibitor und Hilfsstoffe undurchlässige Deckschicht und daran anhaftend eine bis drei Schichten einer Formulierung, die den Phosphodiesterase IV-Inhibitor in bis zu 30 Gew.-% mit bis 70 Gew.-% eines medizinisch akzeptablen Klebers und gegebenenfalls bis 40 Gew.-% eines Penetrationsverstärkers und optional bis 25 Gew.-% Kristallisationsinhibitors enthält. Als medizinisch akzeptable Kleber können beispielsweise Polyacrylat-, Silikonoder Polyisobutylen-Kleber eingesetzt werden. Darüber hinaus sind aber auch Polyurethane, Block-Copolymere auf Styrenbasis und weitere organische Polymere einsetzbar.

Bevorzugt sind Polyacrylatkleber.

Polyacrylat im Sinne des Patentes ist Oberbegriff für alle Polymere (Homo- und Copolymere), die Acrylsäure bzw. Acrylsäurederivate enthalten. Besonders bevorzugt sind Vinylacetat-Acrylat-Copolymere und Acrylat-Vinylpyrrolidon-Copolymere. Am bevorzugtesten sind Heterocopolymere aus Vinylacetat, 2-Ethylhexylacrylat und Hydroxyethylacrylat (Gelva® -MPS 7881 und 7883) sowie Copolymere aus Vinylpyrrolidon und 2-Ethylhexylacrylat (TSR® -Kleber der Firma Sekisui)

Jeder der aufgetragenen Schichten kann ein- oder beidseitig mit einer klebenden Schicht überzogen sein, die zusätzlich penetrationsverstärkende und/oder kristallisationsinhibierende Substanzen enthalten kann.

Weiterhin kann auf die Seite der Formulierung ein Hautaufkleber aufgebracht werden, abdeckend oder am Umfang, die nicht von der undurchlässigen Deckschicht abgedeckt ist. Zum Verpacken und/oder Lagern lässt sich die zugängliche Seite der Formulierung mit einem Trennpapier abdecken.

Zur Herstellung und Aufbringung der Formulierung auf die undurchlässige Deckschicht lässt sich die Formulierung zunächst in flüchtigen Lösungsmitteln ansetzen, wie beispielsweise niederen Alkoholen, Ketonen oder niederen Carbonsäureestem, sowie Ethanol, Isopropanol, Aceton oder Ethylacetat, polaren Ethern, beispielsweise Tetrahydrofuran, niederen Kohlenwasserstoffen, wie Cyclohexan oder Benzin, oder auch Halogenkohlenwasserstoffen, wie Dichlormethan, Trichlormethan, Trichlorfluorethan und Trichlorfluormethan.

Als Penetrationsverstärker können eingesetzt werden:
ein- oder mehrwertige Alkohole wie Ethanol, 1,2-Propandiol oder Benzylalkohol; gesättigte oder ungesättigte Fettalkohole mit 8 bis 18 Kohlenstoffatomen wie Laurylalkohol oder Cetylalkohol; Kohlenwasserstoffe wie Mineralöl; gesättigte und ungesättigte Fettsäuren mit 8 bis 18 Kohlenstoffatomen wie Stearinsäure oder Ölsäure; Fettsäureester mit bis zu 24 Kohlenstoffatomen oder Dicarbonsäurediester mit bis zu 24 Kohlenstoffatomen wie die Methylester, Ethylester, Isopropylester, Butylester, sec.-Butylester, Isobutylester, tert.-Butylester oder Monoglycerinsäureester der Essigsäure, Capronsäure, Laurinsäure, Myristinsäure, Stearinsäure und Palmitinsäure, Phosphatidderivate, wie Lecithin, Terpene, Harnstoff und seine Derivate oder Ether wie Dimethylisosorbid und Diethylenglycolmonoethylether.

Besonders bevorzugte sind Laurylalkohol, 1,2-Propandiol, die Methylester und insbesondere die Isopropylester der Myristinsäure oder Ölsäure, Diisopropyladipat und Diisopropylsebacat, Laurinsäure und Ölsäure, sowie deren Gemische.

In einer besonders bevorzugten Ausführungsform enthält die transdermale Formulierung Kristallisationsinhibitoren, die als Komplexbildner geeignet sind, beispielsweise feste Lösungen mit Wirkstoffen zu bilden, die Grenzflächenlöslichkeit für den Wirkstoff erhöhen und die Neigung des Wirkstoffes zur Rekristallisation nach Entfernen eines Prozesslösungsmittels oder Senkung der Temperatur herabsetzen. Der Zusatz von Kristallisationsinhibitoren ermöglicht es, höhere Wirkstoffbeladungen der Formulierung vorzunehmen, ohne dass sich Wirkstoffkristalle bilden, die nur sehr eingeschränkt für den Stofftransport in die Haut zur Verfügung stehen
Als Kristallisationsinhibitoren sind N-Vinyllactam-Polymere wie N-Vinyl-1-Azacycloheptan-2-on-Homopolymer und N-Vinyl-piperidin-2-on-Homopolymer und insbesondere Polymere des Vinylpyrrolidons, wie Polyvidon (Kollidon® ), oder Co-Polymere des Vinylpyrrolidons mit Vinylacetat (Copovidone) geeignet. Besonders bevorzugt ist ein Copovidon aus 6 Teilen Vinylpyrrolidon und 4 Teilen Vinylacetat (Kollidon® VA 64).

Die erfindungsgemäße transdermal wirksame Formulierung ist dabei geeignet, mit einer einfachen Applikation, d. h. Aufheften auf die Haut, eine einfach anzuwendende Formulierung bereitzustellen. Überdies ist die erfindungsgemäße Formulierung in der Lage, konstantere Plasmaspiegel von Phosphodiesterase IV-Inhibitoren hervorzubringen, als beispielsweise injizierte Wirkstoffformulierungen. In der besonders bevorzugten Ausführungsform vermeidet die erfindungsgemäße Formulierung Konzentrationsspitzen des Wirkstoffes, die in einigen Fällen zu Übelkeit bei Patienten führen können. Weiterhin vermeidet die Anwendung der erfindungsgemäßen Formulierung die erste Leberpassage des Wirkstoffes, durch die die Wirkstoffkonzentration im Plasma vermindert werden kann

Die Erfindung wird nun im Detail durch Beispiele erläutert.
Die Herstellung geeigneter enantiomerenreiner Methylphenyloxazolidinon-Derivate ist in WO 97/15561 beschrieben.

<u>Beispiel 1:</u> Herstellung eines Mesopram-Transdermalsystems mit Dimethylisosorbid als Penetrationsverstärker

10,0 g (R)-(-)-5-(4-Methoxyphenyl-3-propoxy)-5-methyl-2-oxazolidinon wird mit 25,5 g Dimethylisosorbid in 50,0 g 2-Propanol in einem Rundkolben unter Rühren bei 55 bis 60 °C gelöst. Beim Auflösen verdunstetes Lösemittel wird anschließend ergänzt. In einem Rührbecher werden 165,0 g einer Lösung des Klebers 2-Ethylhexylacrylat-N-vinyl-2-pyrollidon-Copolymer in Ethylacetat (TSR® -Kleber der Firma Sekisui) vorgelegt und die zuvor hergestellte Lösung wird unter Rühren zugegeben. Der gesamte Ansatz wird für ca. 30 Minuten mittels Flügelrührer luftblasenfrei geführt. Mit Rakelauftrag wird die erhaltene Mischung auf eine fluorpolymerbeschichtete Polyesterfolie (Scotchpak® 9742) aufgetragen, so dass ein Beschichtungsgewicht von 95,0 bis 105,0 g Trockenmasse pro $m^2$ erhalten wird. Die beschichteten Folien werden bei 75 bis 85 °C im Trockenschrank auf einen Restlösemittelgehalt von < 1-2 g/$m^2$ getrocknet. Nach der Trocknung wird eine Polyester- oder Polyethylenfolie (Cotran 9720® der Fa. 3M; FORKO-Liner der Fa. 4P-Folien) auflaminiert. Die nun beidseitig von Folien eingefasste Wirkstoffformulierung wird mit einer Stanzeinrichtung zu geeigneten Größen ausgestanzt und zur Lagerung in Folienbeutel eingesiegelt.

Beispiel 2: Herstellung eines Mesopram-Transdermalsystems mit Copovidone als Kristallisationsinhibitor (Kleber: Gelva® -MPS)

In einem 1-L-Rundkolben werden 120,0 g Copovidone in 280,0 g 2-Propanol unter Rotation bei 50 bis 70 °C gelöst. In einem 1-L-Rührbecher werden 37,5 g (R)-(-)-5-(4-Methoxyphenyl-3-propoxy)-5-methyl-2-oxazolidinon vorgelegt und mit 375,0 g der propanolischen Copovidonelösung unter Rühren gemischt. Zur Homogenisierung kann die Mischung 20 bis 30 Minuten im Ultraschall behandelt werden. In einem 3-L-Rührbecher werden 1229,5 g einer Kleberlösung eines Heterocopolymerengemisches auf der Basis von Vinylacetat und Ethylhexylacrylat (Gelva® MPS 7881) vorgelegt und mit der wirkstoffhaltigen Lösung versetzt.
Der Ansatz wird mit 2-Propanol auf eine Gesamtmasse von 1800,0 g aufgefüllt und mit einem Flügelrührwerk für ca. 30 Minuten blasenfrei gerührt. Mit einer kontinuierlich arbeitenden Beschichtungsvorrichtung wird eine Trägerfolie zu einem Trockengewicht von 100 ± 5 g/m$^2$ mit der zuvor hergestellten Mischung beschichtet. Die beschichtete Trägerfolie wird in einem zweistufigen Trockentunnel bei ca. 78 bis 82 °C getrocknet und einer Bandgeschwindigkeit von 15 cm pro Minute. Anschließend wird eine Trennfolie auflaminiert und die von Folien beidseitig beschichtete Formulierung aufgerollt. Aus der Rollenware werden mittels einer Stanzeinrichtung runde Transdermalsysteme mit einem Durchmesser von 35,6 mm gestanzt und in luftdichte Beutel (Oxyblock) eingesiegelt.

Beispiel 3: Herstellung eines Mesopram-Transdermalsystems mit 1,2-Propandiol und Laurylalkohol als Penetrationsverstärker

In einem Rührbecher werden 13,5 g 1,2-Propandiol, 1,5 g 1-Laurylalkohol und 5,0 g (R)-(-)-5-(4-Methoxyphenyl-3-propoxy)-5-methyl-2-oxazolidinon vereint und in 200,0 g 2-Propanol unter Rühren gelöst. Zu der Lösung werden 224,0 g einer Lösung des Klebers 2-Ethylhexylacrylat-N-vinyl-2-pyrollidon-Copolymer in Ethylacetat (TSR® -Kleber der Firma Sekisui)) gegeben und mit 2-Propanol auf insgesamt 500,0 g ergänzt. Die Lösung wird bis zur vollständigen Homogenisierung und Blasenfreiheit gerührt. Transdermalsysteme werden wie in Beispiel 2 beschrieben hergestellt und konfektioniert.

Beispiel 4: Herstellung eines Mesopram-Transdermalsystems mit Copovidone als Kristallisationsinhibitor (TSR® -Kleber der Firma Sekisui)

In einem Rührbecher werden 12,51 g Copovidone und 2,50 g (R)-(-)-5-(4-Methoxyphenyl-3-propoxy)-5-methyl-2-oxazolidinon unter Rühren in 15,0 g 2-Propanol gelöst. Dieser Lösung werden 52,45 g einer Lösung des Klebers 2- Ethylhexylacrylat-N-vinyl-2-pyrollidon-Copolymer in Ethylacetat (TSR® -Kleber der Firma Sekisui) zugesetzt und der Ansatz wird mit 2-Propanol auf 90,0 g Gesamtmasse aufgefüllt. Die Lösung wird bis zur vollständigen Homogenisierung und Blasenfreiheit gerührt. Anschließend werden Transdermalsysteme wie in Beispiel 2 beschrieben hergestellt und konfektioniert.

Beispiel 5: Herstellung eines zweischichtigen Mesopram-Transdermalsystems mit Copovidone als Kristallisationsinhibitor (Kleber: Gelva® -MPS 7881)

In einem Rührbecher werden 30,0 g Copovidone und 10,0 g (R)-(-)-5-(4-Methoxyphenyl-3-propoxy)-5-methyl-2-oxazolidinon vorgelegt und in 25,0 g 2-Propanol gelöst. Dieser Lösung werden 160,0 g einer Kleberlösung eines Heterocopolymerengemisches auf der Basis von Vinylacetat und Ethylhexylacrylat (Gelva® MPS 7881) zugesetzt und unter Rühren homogenisiert und blasenfrei gerührt. Der Ansatz wird mit 2-Propanol auf 260,0 g aufgefüllt. Die Mischung wird durch Rakelauftrag auf eine Trennfolie (Scotchpak® 9742) aufgetragen und getrocknet, so dass sich eine Beschichtung 95,0 bis 105,0 g Trockenmasse pro m$^2$ ergibt. Anschließend wird auf die noch zugängliche Oberfläche der Formulierung eine weitere Kleberschicht ohne zusätzlichen Wirkstoff oder Hilfsstoff aufgetragen. Die Schichtdicke dieser Kleberschicht wird auf 10 µm eingestellt. Nach erneuter Trocknung wird eine Trägerfolie auflaminiert. Ausstanzen und Verpacken wird gemäß Beispiel 1 vorgenommen.

Beispiel 6: In-Vitro-Messung des Hautflusses von (R)-(-)-5-(4-Methoxyphenyl-3-propoxy)-5-methyl-2-oxazolidinon durch die Haut der haarlosen Maus.

Folgende erfindungsgemäße Transdermalsysteme zeigten nach vierwöchiger Lagerung bei 25 °C in der mikroskopischen Untersuchung keine Kristallbildung:

Tab. 1

| Formulierung TDS | Mesopram Gew.-% | Klebstoff | Penetrations-verstärker | Copovidon |
|---|---|---|---|---|
| A | 5 | 95 % TSR | ohne | ohne |
| B | 5 | 2,5 % TSR | 12,5 % DMI | ohne |
| C | 5 | 80% TSR | 15%PD/LA(9+1) | ohne |
| D | 10 | 75% TSR | ohne | 15% |
| E | 5 | 80 % Gelva | ohne | 15% |
| TSR® = Hauthaftkleber der Fa. Sekisui (Ethylhexylacrylat-Kleber enthaltend 35 % einpolymerisiertes N-Vinylpyrrolidon; DMI = Dimethylisosorbid; PD = Propandiol; LA = Laurylalkohol; alle Formulierungen wurden im Labormaßstab mit Polyesterfolie (Scotchpack® ) als Trennfolie und Polyethylenfolie (Co Tran® 9720) als Trägerfolie hergestellt und auf eine Fläche von 2 $cm^2$ ausgestanzt. | | | | |

Die Haut männlicher haarloser Mäuse (Stamm MF1 hr/hr Ola/Hsd von Winkelmann, Deutschland) im Alter von 3 bis 4 Monaten wurden zu 3 $cm^2$ ventral und dorsal entnommen und nach Entfernen anhaftenden Fettgewebes in Franz'sche Diffusionszellen montiert. Auf die Hautflächen wurde eine der Formulierungen A bis E aufgebracht; gewebsseitig wurde die Haut von HEPESgepufferter Salzlösung nach Hank, mit 1000 I.E. Penicillin versetzt, in Kontakt gebracht. Diese Akzeptorlösung bestand aus 5,9575 g/L HEPES, 0,35 g/L $NaHCO_3$, 0,1 L HBSS 10x (GIBCO 032-04065, Life Technologies GmbH, Berlin) in destilliertem Wasser.
Von der Akzeptorflüssigkeit wurden in den ersten sechs Stunden in zweistündigen Intervallen und in Stunde 6 bis 54 in achtstündigen Intervallen Proben gezogen. Pro Stunde wurde etwa 1 mL Akzeptorflüssigkeit durch die Diffusionszelle mittels einer Peristaltikpumpe gepumpt. Der gesamte Versuchsaufbau war bei 31 ± 1 °C temperiert.

Die Menge des durch die Hautstücke hindurchgetretenen (R)-(-)-5-(4-Methoxyphenyl-3-propoxy)-5-methyl-2-oxazolidinon wurde mittels eines Radioimmunoassays bestimmt.
Der Durchtritt von (R)-(-)-5-(4-Methoxyphenyl-3-propoxy)-5-methyl-2-oxazolidinon ist in Tabelle 2 dargestellt, wie er in den Franz'schen Diffusionszellen gemessen werden konnte.

Tab. 2:

| Formulierung TDS | mittlerer Fluss über 14 bis 46 h (µg $\cdot cm^{-2} \cdot h^{-1}$) | Maximalfluss (µg $\cdot cm^{-2} \cdot h^{-1}$) | $t_{max}$ (h) |
|---|---|---|---|
| A | 2,18±1,03 | 2,58±1,34 | 26 |
| B | 2,35±0,37 | 2,51±0,17 | 26 |
| C | 3,92±1,73 | 4,70±2,38 | 18 |
| D | 3,27±2,31 | 4,16+3,69 | 34 |
| E | 2,61±0,72 | 3,68±3,30 | 34 |
| Mittelwerte ± Standardabweichung; n=4 | | | |

Fig. 1 zeigt die Zeitverläufe des Mesopram-Flusses durch die Maushaut.

<u>Beispiel 7:</u> Pharmakokinetische Untersuchung am Menschen

Die zuvor beschriebene Formulierung E wurde an zwölf gesunden Männem im Alter von 20 bis 42 Jahren mit normalem Körpergewicht getestet, wobei für 72 Stunden jeweils drei Transdermale Formulierungen von je 10 $cm^2$ mit jeweils 5 mg (R)-(-)-5-(4-Methoxyhenyl-3-propoxyp)-5-methyl-2-oxazolidinon simultan auf dem unteren Rückenbereich appliziert wurden. Nach den 72 Stunden wurde die transdermale Formulierung entfernt und innerhalb einer Woche Auswaschzeit die Konzentration des Wirkstoffes im Serum per RIA bestimmt. Die gemessenen Serumspiegel des Wirkstoffes ergaben einen mittleren transdermalen Stoffstrom von 0,49 ± 0,7 µg/$cm^2$/h, bei maximalen Serumspiegeln von 0,88 ng/mL im Zeitintervall von 29 ± 10 h. Insbesondere wurden plateauartige Verläufe des Serumspiegels an Wirkstoff erhalten, wobei das Plateau nach einem etwa linearen Anstieg in den ersten 18 Stunden bis zur Stunde 75 aufrechterhalten werden konnte und anschließend etwa linear abfiel. Aufgrund der ausgeprägten Plateau-Phase, die nach der Applikation der TDS erzielt wurde, konnten zum Zeitpunkt der Abnahme der TDS nach drei Tagen immerhin

noch Mesopram-Konzentrationen im Bereich von 65±16% der Maximalspiegel gemessen werden. Nach Abnahme der TDS fielen die Serumspiegel mit einer Halbwertszeit von 6,1±2,7 h. Die AUC-Werte sowie weitere pharmakokinetische Parameter finden sich in Tab. 3.

In einem Vergleichsversuch mit intravenöser Gabe von insgesamt 0,2 mg (R)-(-)-5-(4-Methoxyphenyl-3-propoxy)-5-methyl-2-oxazolidinon innerhalb einer Stunde wurde um ein Vielfaches höhere Serumspiegel gemessen, die 3,5 mg/mL betrugen. Eine Stunde nach Beendigung der Infusion fielen die mittleren Serumspiegel auf 1,15±0,44 $ng \cdot ml^{-1}$. Im weiteren Verlauf fielen die Serumspiegel ab, um 4 h nach Beendigung der Infusion eine Höhe von 0,39±0,17 $ng \cdot ml^{-1}$, nach 8 h 0,17±0,09 $ng \cdot ml^{-1}$ und nach 24 h 0,08±0,08 $ng \cdot ml^{-1}$ zu erreichen. Die wesentlichen pharmakokinetischen Parameter nach i.v.-Gabe von Mesopram sind in der Tab. 3 dargestellt. Der Vergleichsversuch mit Infusion wurde an denselben Probanden durchgeführt, wie die transdermale Applikation. Bei fünf von zwölf Probanden wurde die Infusion abgebrochen, da sie unter Übelkeit litten.

Fig. 2 zeigt die Konzentrationszeitverläufe von Mesopram nach transdermaler sowie nach intravenöser Gabe.

Tab. 3:

| Pharmakokinetische Kenngrößen des Mesopram-haltigen TDS E und der i.v.-Referenz ($\bar{x}$ ±s, n=12) | | | |
|---|---|---|---|
| Parameter | | Applikation | |
| | | intravenöse Infusion: 0,2 mg über 1h | transdermale Gabe: 15 mg über 3d (3 TDS E á 10 $cm^2$) |
| $C_{max}$ | ($ng \cdot ml^{-1}$) | 3,6±0,9 | 0,88±0,22 |
| $t_{max}$ | (h) | o.A. | 29±10 |
| AUC | ($ng \cdot h \cdot ml^{-1}$) | 9,0±3,7 | 15,8±5,2[a] |
| $t_{½}$ | (h) | 2,5±0,9 | 6,1±2,7 |
| Cl | ($ml \cdot min \cdot kg^{-1}$) | 5,1±1,8 | - |
| TD | ($mg \cdot d^{-1}$) | - | 0,35±0,12 |
| f | (%) | 100 | 7 |
| $C_{max}$ = maximale Konzentration, $t_{max}$ = Zeitpunkt der Maximalkonzentration, f = absolute Bioverfügbarkeit, AUC = Fläche unter der Serumkurve, TD = Tagesdosis (nach transdermaler Gabe im Mittel), Cl = Clearance, $t_{½}$ = Halbwertszeit der Verteilungsphase, o.A. = ohne Angabe aufgrund der unterschiedlichen Länge der Infusionszeiträume (von 42 bis 60 min); [a] je Tag für insgesamt drei Tage; bei der Berechnung der transdermalen Dosen wurden die individuellen i.v.-Dosen berücksichtigt | | | |

Wie die in der Fig. 2 und der Tab. 3 dargestellten Ergebnisse zeigen, weist das TDS E eine außerordentlich konstante Wirkstoffabgabe über den Zeitraum von 3 d auf. Da die Trageeigenschaften der Formulierung, auf der das Transdermalsystem E beruht, auch eine längere Tragedauer zulassen, ist eine Eignung des erhaltenen Systems zumindest als Twice-a-week-TDS (Tragedauer alternierend 3 Tage und 4 Tage) denkbar. Aufgrund der niedrigen Ausschöpfung des Wirkstoffdepots (innerhalb von drei Tagen wurden nur 7% des Wirkstoffs systemisch absorbiert) ist gegebenenfalls sogar eine Applikation im Sinne eines Once-a-week-TDS denkbar.

**Patentansprüche**

1. Transdermalsystem **gekennzeichnet durch** einen Gehalt an einem Phosphodiesterase IV-Inhibitor.

2. Transdermalsystem nach Anspruch 1, **dadurch gekennzeichnet, dass** der Phosphodiesterase IV-Inhibitor in einer Matrix vorliegt.

3. Trandermalsystem gemäß Anspruch 1 oder 2, **dadurch gekennzeichnet, dass** der Phosphodiesterase IV-Inhibitor (R)-(-)-5-(4-Methoxyphenyl-3-alkoxy)-5-methyl-2-oxazolidinon ist, bei dem die Alkylgruppe 1 bis 5 Kohlenstoffatome enthält.

4. Trandermalsystem gemäß Anspruch 3, **dadurch gekennzeichnet, dass** der Phosphodiesterase IV-Inhibitor (R) -(-)-5-(4-Methoxyphenyl-3-propoxy)-5-methyl-2-oxazolidinon ist.

5. Transdermalsystem gemäß einem der Ansprüche 2 bis 4, **dadurch gekennzeichnet, dass** die Matrix Polyacrylatkleber umfasst.

6. Transdermalsystem nach Anspruch 5, **dadurch gekennzeichnet, dass** der Polyacrylatkleber ein Copolymer von zumindest 2 der folgenden Monomere ist: 2-Ethylhexylhexylacrylat, Hydroxyethylhexylacrylat, Vinylacetat, Vinylpyrrolidon.

7. Transdermalsystem nach Anspruch 6, **dadurch gekennzeichnet, dass** der Polyacrylatkleber ein Copolymer aus 2-Ethylhexylacrylat und Hydroxyethyl-acrylat oder ein Copolymer dieser Monomere mit Vinylacetat und 2-Ethylhexylacrylat-N-vinyl-2-pyrrolidon ist.

8. Transdermalsystem nach einem der Ansprüche 2 bis 7, **gekennzeichnet durch** einen Gehalt an Phosphodiesterase IV-Inhibitor von bis zu 30 Gew.-% in der Matrix.

9. Transdermalsystem nach einem der Ansprüche 2 bis 8, **dadurch gekennzeichnet, dass** die Matrix wenigstens einen Kristallisationsinhibitor umfasst.

10. Transdermalsystem nach Anspruch 9, **dadurch gekennzeichnet, dass** die Matrix wenigstens ein N-Vinyllactam-Polymer wie N-Vinyl-1-Azacycloheptan-2-on-Homopolymer, N-Vinyl-piperidin-2-on-Homopolymer, Polymere des Vinylpyrrolidons wie Polyvidon (Kollidon® ), oder Co-Polymere des Vinylpyrrolidons mit Vinylacetat (Copovidone) umfasst.

11. Transdermalsystem nach einem der Ansprüche 1 bis 10, **gekennzeichnet durch** einen zusätzlichen Gehalt an wenigstens einem der folgenden Penetrationsverstärker:
ein- oder mehrwertige Alkohole wie Ethanol, 1,2-Propandiol oder Benzylalkohol; gesättigte oder ungesättigte Fettalkohole mit 8 bis 18 Kohlenstoff-atomen wie Laurylalkohol oder Cetylalkohol; Kohlenwasserstoffe wie Mineralöl; gesättigte und ungesättigte Fettsäuren mit 8 bis 18 Kohlenstoff-atomen wie Stearinsäure oder Ölsäure; Fettsäureester mit bis zu 24 Kohlenstoffatomen oder Dicarbonsäurediester mit bis zu 24 Kohlenstoff-atomen wie die Methylester, Ethylester, Isopropylester, Butylester, sec.-Butylester, Isobutylester, tert.-Butylester oder Monoglycerinsäureester der Essigsäure, Capronsäure, Laurinsäure, Myristinsäure, Stearinsäure und Palmitinsäure, Phosphatidderivate, wie Lecithin, Terpene, Harnstoff und seine Derivate oder Ether wie Dimethylisosorbid und Diethylenglycolmonoethylether.

12. Transdermalsystem nach Anspruch 11, **gekennzeichnet durch** einen Gehalt an wenigstens einem der folgenden Penetrationsverstärker: Laurylalkohol, 1,2-Propandiol, die Methylester und insbesondere die Isopropylester der Myristinsäure oder Ölsäure, Diisopropyladipat und Diisopropylsebacat, Laurinsäure und Ölsäure, sowie deren Gemische.

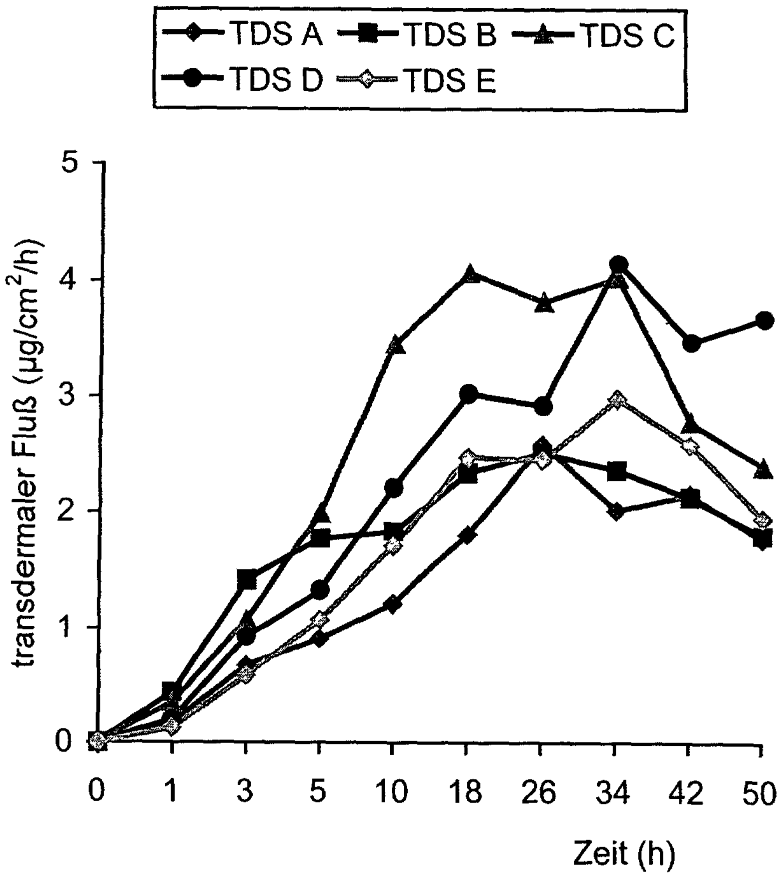
TDS A
TDS B
TDS C
TDS D
TDS E
transdermaler Fluß (µg/cm²/h)
5
4
3
2
1
0
0
1
3
5
10
18
26
34
42
50
Zeit (h)

Fig. 1:

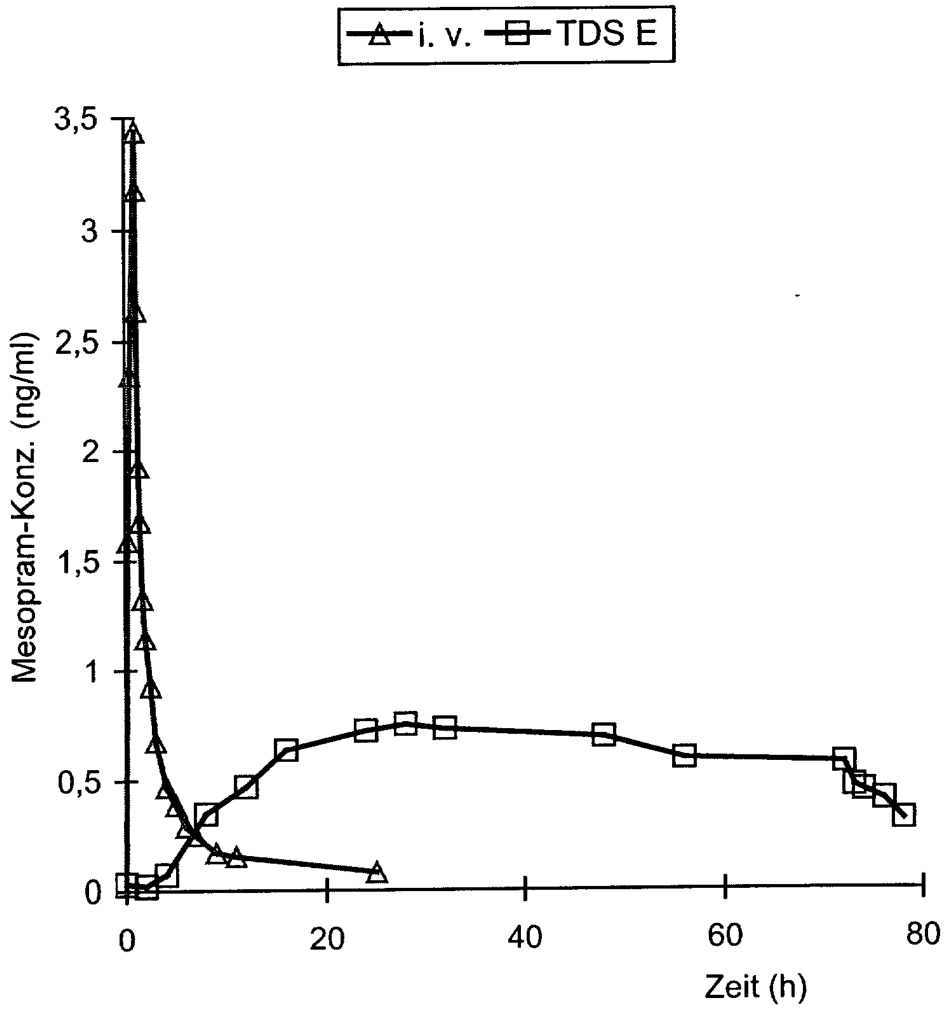


Fig. 2:

Europäisches Patentamt

## EUROPÄISCHER TEILRECHERCHENBERICHT

der nach Regel 45 des Europäischen Patentübereinkommens für das weitere Verfahren als europäischer Recherchenbericht gilt

Nummer der Anmeldung: EP 00 25 0450

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X | DE 39 43 385 A (SCHERING AG) 4. Juli 1991 (1991-07-04) * Beispiel 1 * * Ansprüche * | 1,2,5-8, 11,12 | A61K9/70 A61K31/42 |
| A | DINTER H ET AL: "The type IV phosphodiesterase specific inhibitor mesopram inhibits experimental autoimmune encephalomyelitis in rodents." JOURNAL OF NEUROIMMUNOLOGY, Bd. 108, Nr. 1-2, 1. August 2000 (2000-08-01), Seiten 136-146, XP001001308 ISSN: 0165-5728 * Seite 137, linke Spalte, Zeile 39 * | 1 | |
| D,A | WO 97 15561 A (SCHERING AG) 1. Mai 1997 (1997-05-01) * Seite 1, Absatz 5 * * Seite 5, letzter Absatz - Seite 6, Absatz 4 * * Ansprüche 1-3 * | 1-12 | |

RECHERCHIERTE SACHGEBIETE (Int.Cl.7): A61K

### UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| DEN HAAG | 13. Juli 2001 | Epskamp, S |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C09)

Europäisches Patentamt

**UNVOLLSTÄNDIGE RECHERCHE**
**ERGÄNZUNGSBLATT C**

Nummer der Anmeldung
EP 00 25 0450

Unvollständig recherchierte Ansprüche:
1-12

Grund für die Beschränkung der Recherche:

Die geltenden Patentansprüche 1-12 beziehen sich auf ein Arzneimittel, jeweils charakterisiert durch eine Aktivität: "Phosphodiesterase IV inhibitor". Die Patentansprüche umfassen daher alle Medikamente, die diese Aktivität aufweisen, wohingegen die Patentanmeldung Stütze durch die Beschreibung im Sinne von Art. 83 EPÜ nur für eine begrenzte Zahl solcher Produkte etc. liefert. Im vorliegenden Fall fehlen den Patentansprüchen die entsprechende Stütze bzw. der Patentanmeldung die nötige Offenbarung in einem solchen Maße, daß eine sinnvolle Recherche über den gesamten erstrebten Schutzbereich unmöglich erscheint. Desungeachtet fehlt den Patentansprüchen auch die in Art. 84 EPÜ geforderte Klarheit, nachdem in ihnen versucht wird, das Arzneimittel über das jeweils erstrebte Ergebnis zu definieren. Auch dieser Mangel an Klarheit ist dergestalt, dass er eine sinnvolle Recherche über den gesamten erstrebten Schutzbereich unmöglich macht. Daher wurde die Recherche auf die Teile der Patentansprüche gerichtet, welche im o.a. Sinne als klar, gestützt oder offenbart erscheinen, nämlich die Teile betreffend die Arzeimittel definiert in den Ansprüche 3 und 4. Auch wurde die Recherche gerichtet auf die Konzeption "Phosphodiesterase IV Inhibitor".

## ANHANG ZUM EUROPÄISCHEN RECHERCHENBERICHT ÜBER DIE EUROPÄISCHE PATENTANMELDUNG NR. EP 00 25 0450

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-07-2001

| Im Recherchenbericht angeführtes Patentdokument | Datum der Veröffentlichung | Mitglied(er) der Patentfamilie | Datum der Veröffentlichung |
|---|---|---|---|
| DE 3943385 A | 04-07-1991 | AT 122900 T | 15-06-1995 |
| | | WO 9109634 A | 11-07-1991 |
| | | DE 59009150 D | 29-06-1995 |
| | | DK 460143 T | 09-10-1995 |
| | | EP 0460143 A | 11-12-1991 |
| | | ES 2073731 T | 16-08-1995 |
| | | JP 3034600 B | 17-04-2000 |
| | | JP 4505333 T | 17-09-1992 |
| WO 9715561 A | 01-05-1997 | DE 19540475 A | 24-04-1997 |
| | | AT 201203 T | 15-06-2001 |
| | | AU 715344 B | 20-01-2000 |
| | | AU 4712396 A | 15-05-1997 |
| | | CN 1200115 A | 25-11-1998 |
| | | CZ 9801201 A | 15-07-1998 |
| | | DE 59606920 D | 21-06-2001 |
| | | EP 0859766 A | 26-08-1998 |
| | | FI 980862 A | 17-04-1998 |
| | | HU 9802630 A | 28-04-1999 |
| | | IL 117091 A | 26-07-2000 |
| | | JP 11513693 T | 24-11-1999 |
| | | NO 981688 A | 15-04-1998 |
| | | PL 326322 A | 14-09-1998 |
| | | SK 49598 A | 09-09-1998 |
| | | TR 9800679 T | 21-07-1998 |
| | | US 6025376 A | 15-02-2000 |

EPO FORM P0461

Für nähere Einzelheiten zu diesem Anhang : siehe Amtsblatt des Europäischen Patentamts, Nr.12/82